# EUROPEAN PATENT APPLICATION

(11) **EP 2 656 857 A1**
(43) Date of publication of application: **30.10.2013**
(21) Application number: 12165628.4
(22) Date of filing: 26.04.2012
(51) Int. Cl.: A61K 47/02, A61K 47/12, A61K 9/00, A61K 31/196, A61K 47/48

(54) **Oral liquid formulation**

(71) Applicant: The Jordanian Pharmaceutical Manufacturing Co., 11710 Naor (JO)
(72) Inventor: Qinna, Nidal Adel Mohammad, 11152 Amman (JO); Badwan, Adnan Ali Hussien, 11152 Amman (JO); Al-Akayleh, Faisal Tawfiq, 11732 Amman (JO); Mayyas, Al-Remawi, 13713 Russiefa (JO)
(74) Representative: Scholz, Volker

(57) **Abstract**

The present invention relates to an oral liquid dosage formulation comprising: a) at least one pharmaceutically active compound; b) at least one divalent or trivalent salt; c) at least one pH regulating substance; and d) optionally at least one gelling agent, gelling aid, thickening agent, sweetener, preservative, stabilizing agent, coloring agent, flavoring agent, surfactant and/or emulsifier.

## Description

The present invention relates to a pharmaceutical oral liquid dosage formulation.

Pharmaceutical oral liquid dosage forms are considered as important drug delivery systems for many patients suffering from solid dosage form swallowing problems including geriatrics and pediatrics. However, many drugs exhibit a bad taste and/or are unstable in water which can limit their formulation in a liquid dosage form. There have been attempts in the prior art to formulate water-stable drugs with improved taste by using different techniques.

US 2006/0124695 A1 relates to a liquid drug-containing preparation wherein the pharmaceutically active ingredient is coated by an appropriate coating material, for example a watersoluble cellulose derivative.

US 2006/0182796 A1 discloses a pharmaceutical composition for oral administration containing a pharmaceutically active agent which is coated by a polymeric composition providing solubility in water and stability in an acidic environment. The coating is also used to provide improved taste.

However, due to its core-shell structure, the respective formulations exhibit poor drug release characteristics.

Therefore, it is an object of the present invention to provide a ready-to-use oral liquid dosage formulation which overcomes the drawbacks of the prior art. In particular, an oral liquid dosage formulation shall be provided masking the bad taste which is characteristic for many drugs. Further, it is an object to provide an oral liquid dosage formulation stabilizing drugs which are instable in water and/or acidic media without lowering their release properties.

These objects are achieved by an oral liquid dosage formulation comprising: a) at least one pharmaceutically active compound; b) at least one divalent or trivalent salt; c) at least one pH regulating substance; and d) optionally at least one gelling agent, gelling aid, thickening agent, sweetener, preservative, stabilizing agent, coloring agent, flavoring agent, surfactant and/or emulsifier.

Preferably, the pharmaceutically active compound is an organic acid and/or its salt.

In a preferred embodiment, the pharmaceutically active compound is a non-stereoidal anti-inflammatory drug (NSAID), preferably is selected from the group consisting of Diclofenac sodium, Ibuprofen, Fenbufen, Fenoprofen, Flubifronate, Mephenamic acid, Naproxen and Acetaminophen, most preferably is Diclofenac sodium.

It is preferred that the divalent or trivalent salt is an inorganic divalent or trivalent salt, preferably is selected from the group consisting of magnesium sulfate, magnesium phosphate, magnesium chloride, calcium sulfate, calcium phosphate, calcium chloride, aluminium sulfate, aluminium phosphate and aluminium chloride, most preferably is magnesium chloride.

Also preferred, the pH regulating substance is at least one organic or inorganic acid and/or its salts, preferably is selected from the group consisting of hydrochloric acid, acidic acid, phosphoric acid, sulfuric acid, lactic acid, tartaric acid, malic acid, succinic acid, citric acid and vinegar and/or it salts, most preferably is vinegar.

Furthermore, the pH regulating substance is preferably a buffering system.

In a most preferred embodiment, the pH of the oral liquid dosage formulation is in a range from 3-7, preferably in a range from 4-6, most preferably in a range from 4.5-5.5.

Preferably, the gelling aid is a sodium salt, a potassium salt and/or an ammonium salt.

It is also preferred that the sweetener is selected from the group consisting of saccharine sodium, aspartame, glycerin, sorbitol, sugar, glucose and mannitol.

Most preferably, the preservative is selected from the group consisting of ascorbic acid and sorbic acid and/or its salts.

It is further preferred that the stabilizing agent is selected from the group consisting of ascorbic acid, sodium metaphosphate, butylated hydroxyanisol and butylated hydroxytoluene.

Preferably, the surfactant is preferably selected from the group consisting of polysorbates, sorbitan esters and polyoxyethylene castor oil.

In a most preferred embodiment, the ratio of the at least one pharmaceutically active compound and the at least one divalent or trivalent salt is in a range from 5:1 to 1:5 parts per weight, preferably is in a range from 2:1 to 1:2 parts per weight.

Finally, the ratio of the at least one pharmaceutically active compound to the at least one pH regulating substance is preferably in a range from 10:1 to 1:5 parts per weight, preferably in a range from 5:1 to 1:2 parts per weight, most preferably is in a range from 3:1 to 1:1 parts per weight.

Surprisingly, it was found that the oral liquid dosage formulation according to the present invention solves the problem by masking the bad taste, particularly the bitterness, of many pharmaceutically active compounds and by stabilizing the respective drugs in water and acidic media without diminishing their release properties at the same time.

The inventive oral liquid dosage formulation can, for example, be in a form of a solution, sirup, elixir, emulsion and suspension.

All components of the inventive oral liquid dosage formulation have to be pharmaceutically acceptable. The term "pharmaceutically acceptable" means at least non-toxic.

Further, ingredients used in the inventive formulation shall not negatively influence the taste masking or stabilizing properties of the inventive oral liquid dosage formulation, for example by preventing the formation of the drug-salt-pH-regulating-substance-complex.

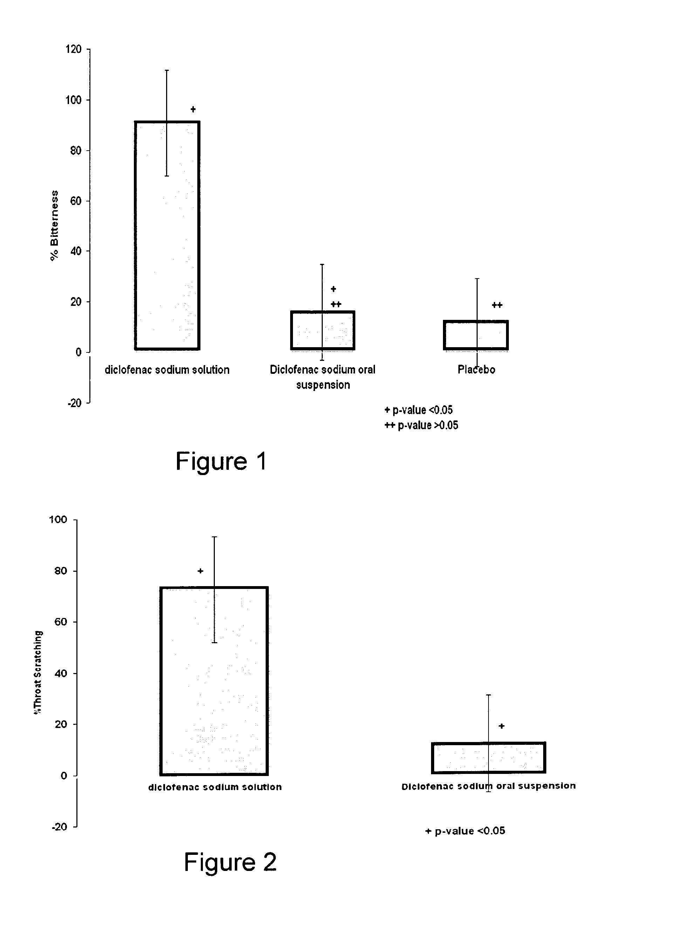

Additional features and advantages of the present invention will become apparent in the following description on the basis of examples with reference to the drawings, wherein
Fig. 1 shows the comparison of bitterness of a aqueous diclofenac sodium solution, diclofenac sodium in the inventive formulation and a placebo.
Fig. 2 shows the comparison of scratching sensation of a aqueous diclofenac sodium solution and diclofenac sodium in the inventive formulation.

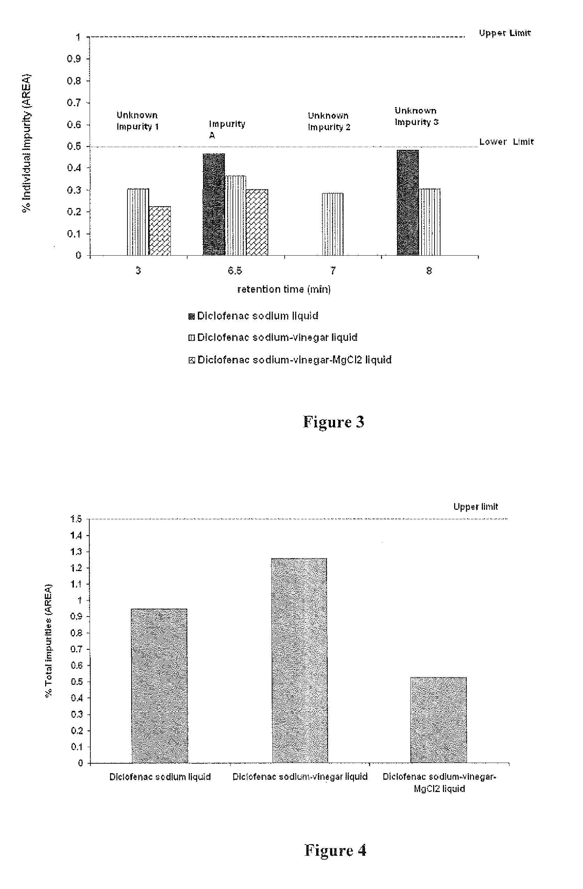

Fig. 3 shows the comparison of the individual impurities of a aqueous solution of diclofenac sodium, a solution of diclofenac sodium and vinegar and a solution of diclofenac sodium, vinegar and magnesium chloride at different retention times.
Fig. 4 shows the comparison of total impurities of a aqueous diclofenac sodium solution, a diclofenac sodium solution and vinegar and a solution of diclofenac sodium, vinegar and magnesium chloride.

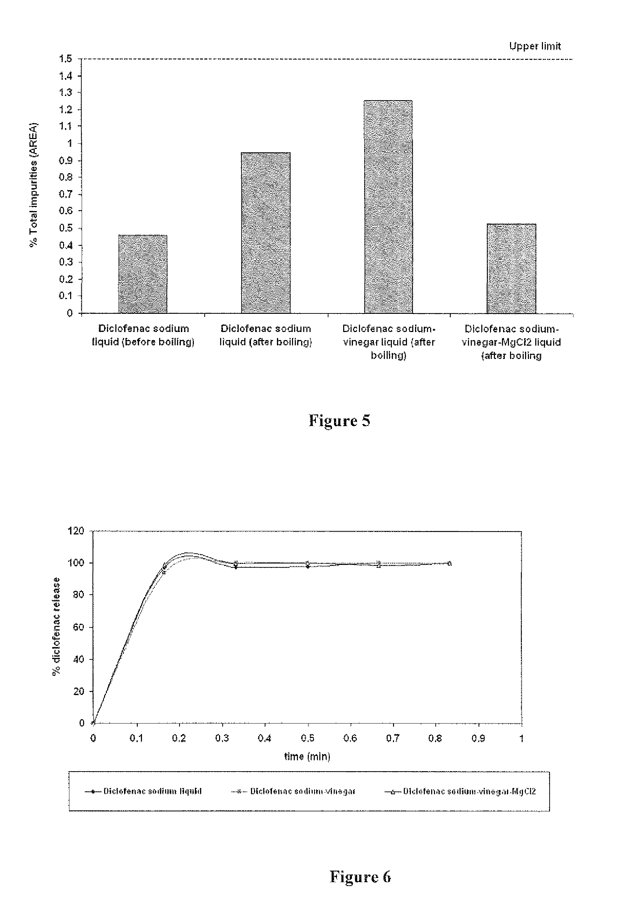

Fig. 5 shows the comparison of the total amount of impurities of different diclofenac sodium containing solutions before and after boiling.
Fig. 6 shows the comparison of the release profile of different diclofenac sodium containing solutions.

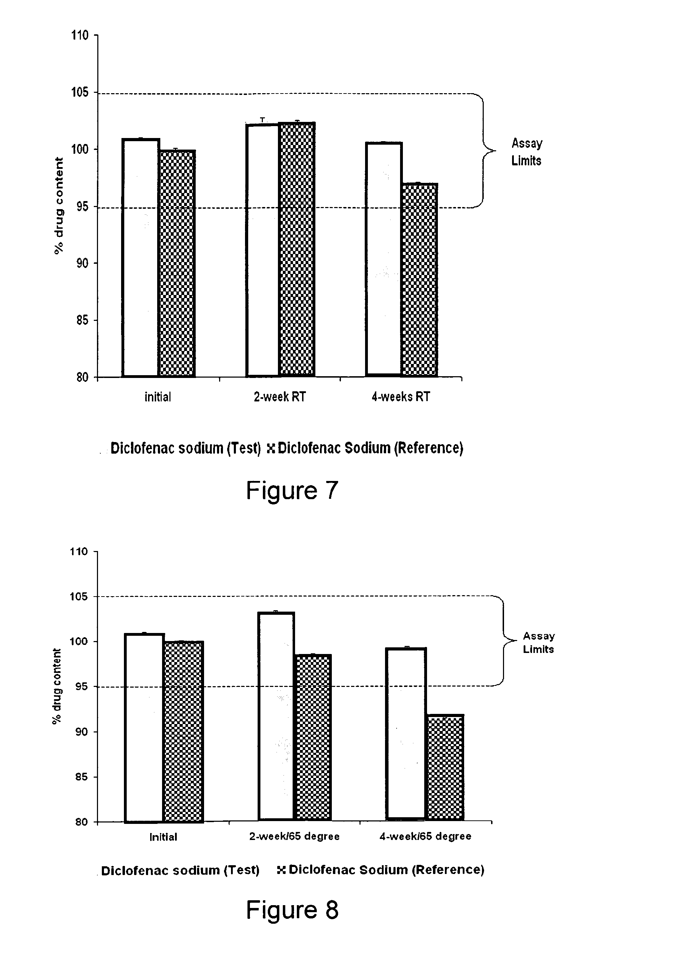

Fig. 7 shows the long-term stability by content of diclofenac sodium in the inventive formulation (test) and in solution (reference) at an initial stage, after two weeks and after four weeks at room temperature.
Fig. 8 shows the long-term stability by content of diclofenac sodium in the inventive formulation (test) and in solution (reference) at an initial stage, after two weeks and after four weeks at 65°C.

### Example 1: Taste mask of the NSAID Diclofenac sodium

Preliminary studies have been done in order to find out how to mask the bitter taste and after taste of Diclofenac sodium (DS) in solution. Different strategies were used in this studies to mask the undesirable taste of the drug. One of the approaches was the formation of a complex of the drug with other chemicals in order to lower the solubility of the drug and to decrease the intensity of bitterness. Another approach was to mask the bitter taste through simple addition of flavors and sweeteners. In a further approach, masking the drug taste shall be achieved by reacting it with different salts. The strategies used to mask the taste of DS solutions are listed below:

### Strategy 1

### Formation of salts with divalent alkali earth metals

The masking effect of divalent alkali earth metal salts (calcium chloride, magnesium chloride and calcium sulphate) was studied by adding solutions of the respective salts dropwise to aqueous solutions of DS as listed in table 1. The resultant solutions were tested for bitter taste.

### Strategy 2

### Use of flavoring agents

The masking effect of different flavoring agents was tested by suppressing the taste of aqueous DS in combination with salts. The resultant solutions were tested for bitter taste and scratching sensation. The bitter taste intensity of the resulting solutions was compared with a pure aqueous solution of DS.

### Strategy 3

### Use of complex forming monosaccharide and polysaccharide compounds

The masking effect of monosaccharide (Glucosamine.HCl) and polysaccharide compounds like chitosan and β-cyclodextrin was evaluated with a view to suppressing the bitterness and scratching sensation of DS. A solution of chitosan was added dropwise to an aqueous solution of DS (Table 1). DS was mixed with β-cyclodextrin in the ratios of 1:1, 1:2, 1:3 and 1:5. The DS solution was added dropwise to the β-cyclodextrin solution while continuously stirring. The resultant solutions were tested for bitter taste and scratching sensation. The bitter taste intensity of the resulting solutions was compared with a pure aqueous solution of DS.

### Strategy 4

### Complex formation with pharmaceutical organic acids

Different Organic acids (citric acid, tartaric acid and lactic acid) were used to determine their masking effect on the bitterness and scratching sensation of DS. Organic acid solutions were added dropwise to an aqueous solution of DS (Table 1). The resultant solutions were tested for bitter taste and scratching sensation. The bitter taste intensity of the resulting solutions was compared with a pure aqueous solution of DS.

**Table 1: Effect of salt, polysaccharide and organic acids in taste mask**

| **Name of ingredient** | **Strategy of masking** | **F1** | **F2** | **F3** | **F4** | **F5** | **F6** | **F7** | **F8** | **F9** | **F10** | **F11** | **F12** |
|---|---|---|---|---|---|---|---|---|---|---|---|---|---|
| **Complex Preparation** | | | | | | | | | | | | | |
| Diclofenac sodium | | 60mg | 60mg | 60mg | 60mg | 60mg | 60mg | 60mg | 60mg | 60mg | 60mg | 60mg | 60mg |
| Calcium chloride 2H2O | S1 | - | 138mg | - | - | - | - | - | - | - | - | - | - |
| Magnesium chloride 6H2O | S1 | - | - | - | - | - | 19mg | - | - | - | - | - | - |
| Calcium sulphate | S1 | - | - | - | - | - | - | - | 27mg | - | - | - | - |
| Glucosamme Hcl | S3 | - | - | - | 406mg | - | | - | - | - | - | - | - |
| Glycine | S3 | - | - | - | - | - | - | - | - | 141 mg | - | - | - |
| Chitosan | S3 | - | - | - | - | - | - | - | - | - | 30mg | - | - |
| Magnesum chloride 6H2O+ Plavors | S1-S2 | - | - | - | - | - | - | - | - | - | - | 19mg+ 015 to 05 %w/v | - |
| Tartaric acid | S4 | - | - | 434mg | - | - | - | - | - | - | - | - | - |
| Lactic acid | S4 | - | - | - | - | 169mg | - | - | - | - | - | - | - |
| Citric acid | S4 | - | - | - | - | - | - | - | - | - | - | - | - |
| Magnesiuin chloride + Citric acid | S1+S4 | - | - | - | - | - | - | 19mg+ 362mg | - | - | - | - | - |
| Magnesium chloride 6H2O+ vinegar (5%) | S1-S4 | - | - | - | - | - | - | - | - | - | - | - | 19mg+ 2%v/v |
| watet | | 30ml | 30ml | 30ml | 30ml | 30ml | 30ml | 30ml | 30ml | 30ml | 30ml | 30ml | 10ml |

| Parameter | | | | | | | | | | | | | |
|---|---|---|---|---|---|---|---|---|---|---|---|---|---|
| **Taste Result** | | Very bitter After taste | Very bitter Rafter taste | Bitter After taste | Bitter After taste | bit^{t}ter After taste | Less bitter After taste | slight bitter After taste | bitter After taste | bitter After taste | bitter After taste | bitter After taste | slight bitter After taste |

For comparison, in formulation F1the drug was not complexed with any compound. F2 relates to the drug-calcium chloride complex in a molar ratio of 2:1. F3 relates to the drug-tartaric acid complex in a molar ratio of 1:1. F4 and F5 relate to the drug-glucosamine.HCl complex and the drug-lactic acid complex, respectively in a molar ratio of 1:1. F6 relates to the drug-Magnesium chloride complex in molar ratio of 2:1. F7 relates to the drug-magnesium choride-citric acid complex in molar ratio of 2:1:1. F8 relates to the drug-calcium sulfate complex in molar ratio of 2:1. F9 and F10 relate to drug-glycine and drug-chitosan complexes, respectively in molar ratios of 1:1. F11 and F12 relate to drug-Magnesium chloride- flavor and drug -magnesium chloride-vinegar complexes.

From all of the complex formulations F7 and F12 featured best improvement in bitter taste masking. F12 was selected as the optimized complex for the further formulation of a suspension. All other formulations were rejected.

**Table 2: Masking effect of vinegar and MgCl₂ on other NSAIDs**

| | **Taste evaluation** | |
|---|---|---|
| **NSAID** | Without MgCl₂ and vinegar | With MgCl₂ and vinegar |
| Diclofenac sodium | Bad | Good |
| Ibuprofen | Bad | Good |
| Fenfufen | Bad | Good |
| Fenoprofen | Bad | Good |
| Flubifronate | Bad | Good |
| Mefenamic acid | Bad | Good |
| Naproxen | Bad | Good |
| Acetaminophen | Bad | Improved |

### Example 2: Diclofenac sodium full formulation procedure

### Preparation of Drug Complex:

Magnesium chloride hexahydrate was dissolved in water. Diclofenac sodium was added to water and dissolved by stirring. The solution of Diclofenac sodium was added dropwise to the magnesium chloride solution under stirring. Vinegar (5%v/v acid) was added dropwise to the mixture under stirring. Afterwards polysorbate 80 (1%v/v) was added to the mixture under stirring. The liquid obtained by stirring was collected and used for the further preparation of a suspension.

### Preparation of syrup base:

Sugar was dissolved in boiling water. Glycerine, sorbitol, xanthane gum and Carbopol 971NF were added to the sugar solution under homogenization.

### Mixing of Drug complex with syrup:

The obtained drug complex was added to the syrup base under homogenization. A preservative (sodium benzoate), coloring and flavouring agents were added to the above solution and homogenized. The volume of the suspension was made up to the required volume using purified water.

**Table 3: Optimized formula of DS oral suspension**

| ***Diclofenac-Sodium Suspension*** | | | |
|---|---|---|---|
| ***S*/*N*** | ***Ingredient*** | ***g*/*500ml*** | ***W*/*V%*** |
| 1 | Diclofenac-Sodium | 125gm | 025% |
| 2 | Magnesium chloride | 1 2gm | 0 24% |
| 3 | Vinegar (5% acidity) | 10ml | 0 002% |
| 4 | Tween 80 | 20drops | 0 0016% |
| 5 | Sucrose | 150 | 30% |
| 6 | Xanthan-gum | 075 | 0 15% |
| 7 | Glycerin | 125 | 25% |
| 8 | Sorbitol | 25 | 5% |
| 9 | Na Benzoate | 0 5 | 0 1% |
| 10 | Carbopol 971NF | 1 25 | 0 25% |
| 11 | Sunset yellow | 0 05 | 0 01% |
| 12 | Orange flavor | 3ml | 06v/v% |
| 13 | water | q.s 500ml | q s 100% |

### Example 3:Taste evaluation

Taste evaluation was performed with the participation of well informed healthy volunteers (women and men aged 20-60 years). To train and assess the ability of the volunteers in detecting the intensity of bitterness, standard chloroquine solutions of concentrations of 0.08mg/ml, 0.4mg/ml and 2mg/ml were used. The subjects were asked to keep the standard solutions in their mouth for few seconds and to subsequently rate the bitterness intensity of each standard concentration solution on a numerical scale.

After tasting the standard solutions, the volunteers were asked to taste an unmasked sample of aqueous Diclofenac sodium solution (reference), an inventive masked Diclofenac sodium suspension and a placebo. Samples of 2-3ml were given. The subjects scored the intensity of bitterness/sweetness by placing the given amount of a sample in the tongue for a few seconds to provide time for evaluation. Each panelist judged the taste of the samples based on numerical scale, assessing each solution or suspension according to its degree of sweetness or bitterness and/or scratching sensation. After evaluating the samples the subjects gargled their mouths carefully with water and waited for at least 20 minutes before tasting the next sample. Scaling ranged from 1-5 (1- extremely bitter, 2-very bitter, 3-moderately bitter, 4-slightly bitter, 5- no bitterness). Scaling to assess scratching sensation ranged from 1-5 (1-extreme scratching, 2-high scratching, 3-moderate scratching, 4-slight scratching, 5-no scratching). Scaling to assess sweetness ranged from 1-5 (1-no sweetness, 2-slightly sweet, 3-moderately sweet, 4-highly sweet, 5 extremely sweet).

### Taste evaluation results:

### Example 4: Compatibility study

DS is mentioned in the litrature to be unstable in acidic media. In this study, DS solutions were prepared in a pH range from 4.5-5.5. To determine the stability of the drug when mixed with the masking ingredients, a compatitbility study was done. Compatability of the drug was measured by exposing the liquid formualtions to extreme condition. Prepared formulae were exposed to 200°C under reflex.

### Procedure:

### Diclofenac-sodium solution (F1) -not exposed to boiling

Diclofenac sodium (250.0 mg) was dissolved in 100 ml purified water and pH of the solution adjusted to 4.5 using 0.1M HCl. The solution was diluted to 500 ml volume with methanol. The sample was analyzed by high performance liquid chromatography (HPLC) USP method with modification in diluent. The chromatographic condition are listed in table 4.

**Table 4: Chromatographic conditons**

| **Paramter** | **Condition** |
|---|---|
| chromatography | Shimadzu- HPLC |
| column | 4.6mmx25cm column (L7) hypersil BDS, C8 |
| Mobile phase | Methanol and phosphate buffer (700:300). |
| | Filtered through 0.45µ filter paper, degassed and sonicated |
| Injection volume | 10 µL |
| Detection | 254 nm |
| Temperature | ambient |
| Flow rate | 1ml/min |

### Diclofenac-sodium solution (F2) -exposed to boiling

Diclofenac sodium (250 mg) was dissolved in 100ml purified water and the pH of the solution was adjusted to 4.5 using 0.1M HCl. The mixture was heated for 1 hour to 200°C under reflux. Afterwards, the solution was diluted to 500ml with methanol. The sample was analyzed using HPLC USP method. The same chromatographic conditions were used as listed in table 4.

### Diclofenac-sodium-vinegar liquid (F3) -exposed to boiling

DS (250.0 mg) was dissolved in 50 ml purified water. 30 ml of vinegar (0.5% acidity) were added dropwise to the DS solution. The mixture was boiled for 1 hour under reflux. The liquid was cooled, diluted and completed to 500 ml volume with methanol. The sample was analyzed using HPLC USP method with modification in diluent. The same chromatographic conditions were used as listed in table 4.

### Diclofenac-sodium-MgCl₂-vinegar liquid (F4) -exposed to boiling

Magnesium chloride hexahydrate (79.6 mg) was dissolved in 20 ml purified water. Next 250 mg Diclofenac sodium was separately dissolved in 50.0 ml purified water under stirring. Diclofenac sodium solution was added dropwise to the magnesium chloride solution. Finally 30.0 ml of 0.5% acidity vinegar were added dropwise to the mixture of Diclofenac sodium and magnesium chloride solution. The mixture was then boiled for one hour at 200°C under reflux. The sample was cooled, diluted and completed to 500 ml volume. The sample was placed in HPLC for analysis as per USP version monograph with modification in diluent. The same chromatographic conditions were used as listed in table 4.

**Table 4: composition of DS liquid formulations tested for compatibility study**

| **Permutation code** | **Ingredients** | | | | **Parameter** | |
|---|---|---|---|---|---|---|
| | Diclofenac-sodium | Magnesium chloride 61I20 | Vinegar (0 5%acidity) | Purified water | **Condition** | **pH** |
| F1 | 250mg | | | 100ml | Not exposed to boiling | Range from 4 5-5 5 |
| F2 | 250mg | | | 100ml | Exposed to broiling | |
| F3 | 250mg | | 30ml | 70ml | Exposed to boiling | |
| F4 | 250mg | 79 6mg | 30ml | 70ml | Exposed to boiling | |

### Example 5: In vitro release (full formula)

*In vitro* drug release of the suspension was tested using dissolution apparatus (Paddle type). Five milliliter (12.5 mg) of the DS suspension were given to 600 ml of the dissolution medium (phosphate buffer 6.8 pH) and stirred at 100 rpm at 37°C. Aliquots of the medium were withdrawn at predetermined time intervals (10, 20, 30, 40, 50 and 60) and equivalent amounts of fresh medium were added. The collected samples were analyzed by UV spectroscopy at a wavelength of 275 nm using phosphate buffer as blank to determine the percentage of Diclofenac-sodium release.

### Standard preparation

DS (20.8 mg) was weighed and dissolved in 1000 ml phophate buffer of pH 6.8.

### Phosphate buffer reparation

Potassium dihydrogen phosphate (13.6 g) was dissolved in 2000 ml purified water. The buffer was adjusted to pH 6.8 with NaOH.

### Example 6: Stability test (full formula)

The DS suspension and reference samples were filled in 60 ml amber glass bottles. The filled amber glass bottles were placed in stability chamber maintained at 65°C and at room temperature for a period of 1 month.

Stability of the DS oral suspension was tested for a 1-month storage period. Each week of the storage, the composition of the said suspensions was analyzed by HPLC chromatography for the amount of Diclofenac sodium and its impurities. The same chromatographic conditions were used as listed in table 4.

### Impurity A primary standard preparation:

2,6 dichlorophenyl indoline (20 mg) was dissolved in 100 ml methanol. 0.75 ml of dissolved solution was taken and diluted to 100 ml volume with methanol.

### Sample preparation:

5 ml of test formula/reference samples were pippeted and placed in a 50 ml volumetric flask. The sample was diluted with methanol to 50 ml volume and filtered using syringe filter. 32 ml of the filtrate were taken and diluted to 50 Il with methanol. Samples were analyzed by HPLC chromatography as per USP version monograph of DS tablet with modification in diluent. The same chromatographic conditions were used as listed in table 4.

The features disclosed in the foregoing description, in the claims and in the accompanying drawings may, both separately and in any combination, be material for realizing the invention in diverse forms thereof.

## Claims

1. Oral liquid dosage formulation comprising:
a) at least one pharmaceutically active compound;
b) at least one divalent or trivalent salt;
c) at least one pH regulating substance; and
d) optionally at least one gelling agent, gelling aid, thickening agent, sweetener, preservative, stabilizing agent, coloring agent, flavoring agent, surfactant and/or emulsifier.

2. Oral liquid dosage formulation according to claim 1, wherein the pharmaceutically active compound is an organic acid and/or its salt.

3. Oral liquid dosage formulation according to claim 1 or 2, wherein the pharmaceutically active compound is a non-stereoidal anti-inflammatory drug (NSAID), preferably is selected from the group consisting of Diclofenac sodium, Ibuprofen, Fenbufen, Fenoprofen, Flubifronate, Mefenamic acid, Naproxen and Acetaminophen, most preferably is Diclofenac sodium.

4. Oral liquid dosage formulation according to any of the preceding claims, wherein the divalent or trivalent salt is an inorganic divalent or trivalent salt, preferably is selected from the group consisting of magnesium sulfate, magnesium phosphate, magnesium chloride, calcium sulfate, calcium phosphate, calcium chloride, aluminium sulfate, aluminium phosphate and aluminium chloride, most preferably is magnesium chloride.

5. Oral liquid dosage formulation according to any of the preceding claims, wherein the pH regulating substance is at least one organic or inorganic acid and/or its salts, preferably is selected from the group consisting of hydrochloric acid, acidic acid, phosphoric acid, sulfuric acid, lactic acid, tartaric acid, malic acid, succinic acid and citric acid, vinegar and/or it salts, most preferably is vinegar.

6. Oral liquid dosage formulation according to any of the preceding claims, wherein the pH regulating substance is a buffering system.

7. Oral liquid dosage formulation according to any of the preceding claims, wherein the pH of the oral liquid dosage formulation is in a range from 3-7, preferably in a range from 4-6, most preferably in a range from 4.5-5.5.

8. Oral liquid dosage formulation according to any of the preceding claims, wherein the gelling aid is a sodium salt, a potassium salt and/or an ammonium salt.

9. Oral liquid dosage formulation according to any of the preceding claims, wherein the sweetener is selected from the group consisting of saccharine sodium, aspartame, glycerin, sorbitol, sugar, glucose and mannitol.

10. Oral liquid dosage formulation according to any of the preceding claims, wherein the preservative is selected from the group consisting of ascorbic acid and sorbic acid and/or its salts.

11. Oral liquid dosage formulation according to any of the preceding claims, wherein the stabilizing agent is selected from the group consisting of ascorbic acid, sodium metaphosphate, butylated hydroxyanisol and butylated hydroxytoluene.

12. Oral liquid dosage formulation according to any of the preceding claims, wherein the surfactant is selected from the group consisting of polysorbates, sorbitan esters and polyoxyethylene castor oil.

13. Oral liquid dosage formulation according to any of the preceding claims, wherein the ratio of the at least one pharmaceutically active compound and the at least one divalent or trivalent salt is in a range from 5:1 to 1:5 parts per weight, preferably is in a range from 2:1 to 1:2 parts per weight.

14. Oral liquid dosage formulation according to any of the preceding claims, wherein the ratio of the at least one pharmaceutically active compound to the at least one pH regulating substance is in a range from 10:1 1 to 1:5 parts per weight, preferably in a range from 5:1 to 1:2 parts per weight, most preferably is in a range from 3:1 to 1:1 parts per weight.
